# EUROPEAN PATENT SPECIFICATION

(11) **EP 0 365 655 B1**
(45) Date of publication and mention of the grant of the patent: **21.09.1994**
(21) Application number: 89905288.0
(22) Date of filing: 14.04.1989
(51) Int. Cl.: A61F 2/02, A61F 2/06, A61K 37/02, D02G 3/00, C07K 5/02

(54) **BIOELASTOMERIC MATERIALS SUITABLE FOR THE PROTECTION OF WOUND REPAIR SITES FROM THE OCCURRENCE OF ADHESIONS**
BIOELASTOMER OHNE ADHÄSIONSEIGENSCHAFT ZUM EINSATZ AM WUNDORT
MATERIAUX BIOELASTOMERES ADAPTES POUR EMPECHER QUE DES ADHERENCES NE SE PRODUISENT SUR DES SITES DE REPARATION DE BLESSURE

(30) Priority: 21.04.1988 US 184407
(43) Date of publication of application: 02.05.1990
(73) Proprietor: UAB RESEARCH FOUNDATION, Birmingham, Alabama 35294-2010 (US)
(72) Inventor: URRY, Dan, W., Birmingham, AL 35216 (US)
(74) Representative: Harrison, David Christopher
(86) International application number: US8901482
(87) International publication number: WO8910099

(56) References cited:
- WO-A-87/06238
- US-A- 4 132 746
- US-A- 4 474 851
- US-A- 4 500 700
- US-A- 4 589 882
- US-A- 4 605 413
- US-A- 4 783 523
- BIOPOLYMERS, vol. 25, 1986, pages 1939-1953, John Wiley & Sons, Inc.; D.W. URRY et al.: "Temperature-correlated force and structure development in elastomeric polypeptides: The Ile analog of the polypentapeptide of elastin"
- BIOMAT., MED. DEV., ART. ORG., vol. 9, no. 3, 1981, pages 181-194, Marcel Dekker, Inc.; D.W. URRY et al.: "Compounding of elastin polypentapeptide to collagen analogue: A potential elastomeric prosthetic material"
- BIOCHEMICAL AND BIOPHYSICAL RESEARCH COMMUNICATIONS, vol. 90, no. 1, 12th September 1979, pages 194-198, Academic Press, Inc.: D.W. URRY et al.: "Prolyl hydroxylation of the polypentapeptide model of elastin impairs fiber formation"
- POLYM. PREPR. (AM. CHEM. SOC. DIV. POLYM. CHEM.) vol. 24, no. 1, 1983, pages 3-4; D.W. URRY et al.: "A new class of elastomeric biomaterials: dynamic beta-spirals comprised of sequential polypeptides"

## Description

This invention relates to bioelastic materials which are suitable for the protection of burn areas or the protection of wound repair sites from the development of debilitating adhesions.

Adhesions accompanying the healing of wounds, whether due to surgery or other trauma, give rise to many disadvantageous effects. For example, peritoneal cavity adhesions lead to intestinal obstruction and necessitate recurring operations. Furthermore, unwanted adhesions themselves pose problems during recurrent operations. For example, tendon adhesions often compromise tendon surgery and repair. Clearly, it would be extremely advantageous to find a suitable material that could function as an insulator material isolating wound repair sites from adhesions whether between layers of abdominal and thoracic walls, between repair sites within the abdomen or thorax, within the hand, wrist, foot, ankle, and other joints or between the skin and body stroma. However, such a material would have to satisfy many prerequisites. For example, it would be necessary that such a material would match the compliance of the soft tissue site of application. The material would also need to be biodegradable and be obtainable in different forms, such as elastomeric sheets, foams or powders, that would provide sufficient ease of handling for each particular application. Of course, such a material would also have to be readily sterilizable as well as being biocompatible and eliciting insignificant immunogenic and antigenic responses in the host.

The same properties described above would also be advantageous in a material for the protection of burn areas and to facilitate repair of the damaged tissue.

WO-A-8 706 238 discloses polypeptide bioelastomers which can be used as replacements for elastin and are for use in prosthetic devices.

US-A-4 605 413 discloses chemotactic peptides comprising repeating elastin hexapeptide units for use in conjunction with prosthetic devices.

The concept of the present invention is based on a synthetic elastomeric polypeptide material, capable of reversibly contracting and relaxing by an inverse temperature transition, in a form suitable for use as a protective layer between a mammalian repair site and a second tissue site, the elastomeric material being capable of substantially preventing adhesion between the repair site and the second tissue site; and accordingly relates to the use of such elastomeric material for the manufacture of a barrier for prevention of adhesion between a mammalian wound repair site and a second tissue site.

### In the Drawings:

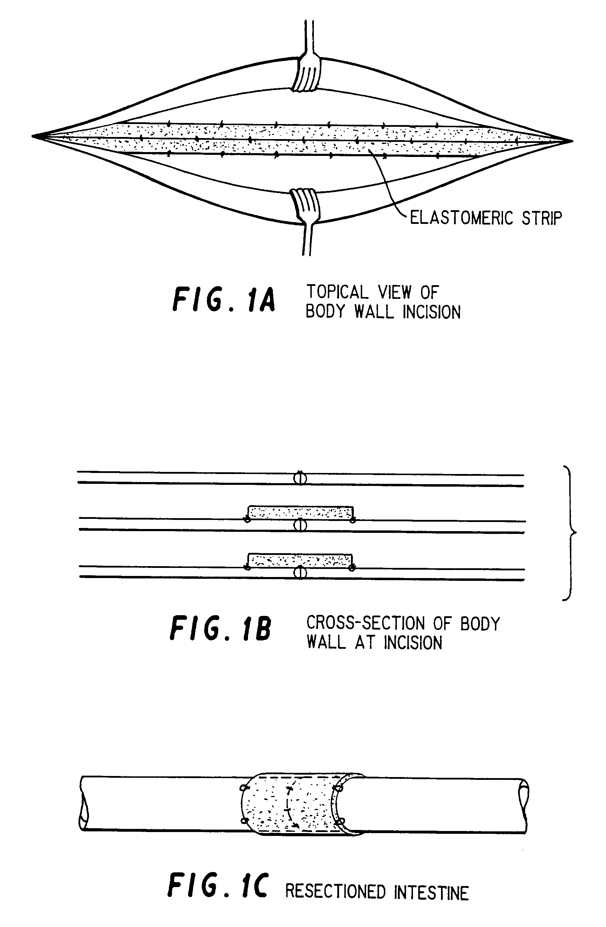

Figure 1(a) is a topical view of a body wall incision covered with an elastomeric strip of the present invention.
Figure 1(b) is a cross-section of a body wall incision with the sutured sites of each layer separated one from the other by elastomeric strips.
Figure 1(c) is an illustration of a resectioned intestine, the sutured site of which is protected by a sleeve of the present elastomeric material.

The present invention uses elastomeric polypentapeptides such as (VPGVG)ₙ as a matrix which may then be modified in a variety of ways to obtain particular properties. For example, the polyhexapeptide (VAPGVG)ₙ may be added either in parallel or in sequence to increase strength, elastic modulus and ease of handling. Furthermore, for specific applications, cell attachment sequences for appropriate tissue cells, may be added covalently and sequentially to provide for the desired cell adhesion as in a tendon sheath or in a fascia or in burn covers.

The elastomeric polypeptides have the ability to control adhesions at wound repair sites. Under physiological conditions, the present biomaterials contain an effective amount of an elastomeric polypeptide and water. Moreover, the present polypeptides are biodegradable, biocompatible and are readily sterilizable. Furthermore, the present biomaterials may be formed in cross-linked sheets or strips varying from a gelatinous to a teflon-like consistency and it is even possible to prepare these materials in a deformable foam-like state with or without cross-linking. Also when efficacious the sequential polypeptide could be applied as a powder which on absorbing water is a sticky viscoelastic gel-like material.

Of particular interest are the polypeptides containing repeating peptide sequences that occur naturally within the elastic fiber of biological connective tissue. The primary sequential polypeptides are (VPGVG)ₙ and (VAPGVG)ₙ. The polypentapeptide is biocompatible and biodegradable as a soft tissue implant and, also as a natural component of connective tissue. The same is true for the polyhexapeptide. The biomaterial is sterilizable as it withstands autoclaving conditions and is cross-linkable to form elastomeric matrices by γ-irradiation at 10-30 Mrad. By varying the proportions of polypentapeptide and polyhexapeptide in the bioelastomer as well as the Mrad dosage, the elastic modulus may be varied and the material consistency can be changed from gelatinous to teflon-like. In addition to cross-linking by γ-irradiation, other means of cross-linking may be used so long as they are biocompatable.

### I. The Elastomeric Materials

The elastomeric materials which can be utilized in the present invention are disclosed and discussed in US-A-4898926 (column 7 onwards) and 4870055 (column 3 onwards). US-A-4898926 corresponds to EP-A-377567 and US-A-4870055 to WO-A-87/06238.

In addition to the particular sequences noted above, other sequences of amino acids may be incorporated in the elastomeric material of the present invention without limitation provided that the material so made retains elasticity and exhibits an inverse temperature transition and the properties associated therewith.

In particular, and for example, while the hexameric sequence described above (̵APGVGV)̵, used to enhance the mechanical properties of a bioelastomer containing the same, is most often used, other hexameric sequences may also be used. In the sequence (̵APGVGV)̵, instead of A, Val, Ile, Phe and Leu may be used. However, it is preferred that P be proline. Instead of P, Val, Ile, Phe, Leu or even Ala may be used. Instead of the first G, Ala may be used. Instead of the first V, Phe, Leu, Ile or Ala may be used. Instead of the second V, Phe, Leu, Ile, Val or Ala may be used.

Notably, occasional substitutions with the full range of known and available amino acids may be used with the proviso that the biocompatibility, biodegradability and elastomeric properties be maintained as has been disclosed in US-A-4898926.

Furthermore, in synthesizing the polypeptides, since all of the amino acids present have corresponding DNA codons, the polypeptides can be produced by genetic engineering using synthetic genes containing codons that correspond to the desired amino acid sequence.

### II. The Use of the Present Elastomeric Materials for the Isolation of Wound Repair Sites in the Prevention of Adhesion Between a Damaged or Repaired Site and a Second Site.

The elastomeric materials may be formed as strips and used as such, or the present elastomeric materials may be applied to the areas of wound repair sites such as incision sites or burn sites as powders, gels or foams.

If the elastomeric materials are applied to an incision, their application may appear as illustrated in Figure 1. In particular, the elastomeric strip is applied to the surface of the incision and stitched thereto and the next layer would then be closed.

The present elastomeric materials may be applied, however, as powders, gels or foams, as for example in body cavities such as the abdomen and thorax. As a foam, the present elastomeric materials may be formulated similar to those disclosed in US-A-3,969,498 and 4,495,168.

Also, the present elastomeric materials may be applied as a gel. As such, they may be formulated similar to the gels disclosed in US-A-4,291,025 and 4,393,048.

Additionally, the present elastomeric materials may also be applied as a powder to an incision site or wound repair area in a manner similar to that disclosed in US-A-4,287,177.

In general, and regardless of whether the elastomeric materials are applied as strips, ribbons or sheets or even as powders, gels, or foams, the rate at which the materials biodegrade will be determined, in part, by the thickness of the material applied. That is, applications of lesser amounts of powders, gels or foams and thinner strips, ribbons or sheets will result in a protective adhesion-preventing layer which will biodegrade at a relatively quicker rate. The converse will be true for thicker layers.

Additionally, it is also possible to slow the rate of biodegradation by incorporating D-amino acids in the peptide sequences or by incorporating L- or D-amino acids containing sterically large side-chains which would tend to inhibit enzymatic hydrolysis of the peptide bonds.

The elastomeric materials may be applied with the ordinary skill of one performing surgery. In applying the present materials, an effective quantity of elastomeric material is applied in any desired form to the site undergoing repair. If the material is applied as a strip or ribbon or sheet, it may be conveniently sutured over an incision site or a burn area or any skin injury. If the material is added as a powder, which expands upon hydration, or a foam or gel, the material may either be sutured over the incision site or applied and left unsutured. Thereafter, the incision of the next layer is then sutured. As already noted, this places the elastomeric material as an elastic barrier separating the repair sites such that connective tissue will not form between the incisions of each layer or between repairing sites within a body cavity.

Generally, regardless of whether the elastomeric materials are applied as a gel, foam or powder or strip, as an approximation, as little as 1 mg or as much as 100 grams of material may be required within a body cavity or on the surface of an incision site or burn area or other skin injury or abrasion. If the elastomeric materials are applied as a sheet, ribbon or strip, or even as gels, foams or powders, a thickness of from about 0.0004 to 0.004 cm (0.001 inches to 0.1 inches) will generally be used. However, the thickness may be more or less as is needed.

Further, the present materials are preferably used in the cross-linked state as, thereby, they will have greater durability and ease of handling. The strips and ribbons of the present invention are used in a cross-linked state, whereas the foams, gels and powders are used in an uncrosslinked states.

The elastomeric materials may be advantageously used in the protection of wound sites in humans against adhesion to second tissue sites. However, they may also be so used with other mammals such as dog, cats, horses, cows or other farm and domestic animals in veterinary medicine.

## Claims

1. The use of a synthetic elastomeric polypeptide material, capable of reversibly contracting and relaxing by an inverse temperature transition, for the manufacture of a barrier for prevention of adhesion between a mammalian wound repair site and a second tissue site.

2. The use of an elastomeric material according to claim 1, wherein the elastomeric material comprises a bioelastomer which has elastomeric tetrapeptide and/or pentapeptide repeating units comprising hydrophobic amino acid and glycine residues, the repeating units existing in a conformation having a β-turn, the bioelastomer having a polypentapeptide repeating unit of the formula:
(̵R₁PR₂R₃G)̵ₙ
wherein R₁ is a peptide-producing residue of Phe, Leu, Ile or Val; R₂ is a peptide-producing residue of Ala or Gly; R₃ is a peptide-producing residue of Phe, Leu, Ile or Val; P is a L-proline-producing residue, and G is a glycine-producing residue, and n is an integer from 1 to 5000.

3. The use of an elastomeric material according to claim 2 wherein R₁ is a peptide-producing residue of L-isoleucine or of L-valine; R₂ is a peptide-producing residue of glycine; and R₃ is a peptide-producing residue of L-valine.

4. The use of an elastomeric material according to claim 1, wherein the elastomeric material comprises a bioelastomer which has elastomeric tetrapeptide and/or pentapeptide repeating units comprising hydrophobic amino acid and glycine residues, the repeating units existing in a conformation having a β-turn, the bioelastomer having
(A) a pentapeptide repeating unit of the formula IPGVG in a polypentapeptide unit of the formula:
-X¹-(IPGVG)ₙ-Y₁-
and
(B) a pentapeptide repeating unit of the formula VPGVG in a polypentapeptide unit of the formula:
-X²-(VPGVG)ₙ-Y²-
wherein
I is a peptide-forming residue of L-isoleucine;
P is a peptide-forming residue of L-proline;
G is a peptide-forming residue of glycine;
V is a peptide-forming residue of L-valine; and
wherein X¹ and X² are each PGVG, GVG, VG, G or a covalent bond; Y¹ is IPGV, IPG, IP, I or a covalent bond; and n in both formulas is an integer from 1 to 200, or n is 0, X¹ and Y¹ or X² and Y² together constituting at least one of said pentapeptide repeating units IPGVG or VPGVG.

5. The use of an elastomeric material according to claim 1, wherein the elastomeric material comprises a bioelastomer which has elastomeric tetrapeptide and/or pentapeptide repeating units comprising hydrophobic amino acid and glycine residues, the repeating units existing in a conformation having a β-turn, the bioelastomer having a polytetrapeptide repeating unit of the formula:
(̵R₁PGG)̵ₙ
wherein R₁ is a peptide-producing residue of Phe, Leu, Ile or Val; P is a L-proline-producing residue, and G is a glycine-producing residue; and n is an integer from 1 to 5000.

6. The use of an elastomeric material according to claim 5 wherein R₁ is a peptide-producing residue of L-valine or of L-isoleucine.

7. The use of an elastomeric material according to any one of claims 2 to 8 wherein the bioelastomer has a hexapeptide repeating unit of the formula APGVGV in a polyhexapeptide unit of the formula
-X(̵APGVGV)̵ₙY-
wherein
A is a peptide-forming residue of L-alanine;
P is a peptide-forming residue of L-proline;
G is a peptide-forming residue of glycine;
V is a peptide-forming residue of L-valine;
and X is PGVGV, GVGV, VGV, GV, V or a covalent bond; Y is APGVG, APGV, APG, AP, A or a covalent bond; and n is an integer of from 2 to about 5000, wherein the unit contains at least 18 amino acid residues.

8. The use according to any one of claims 1 to 7 wherein the elastomeric material is substantially crosslinked.

9. The use according to any one of claims 1 to 7 wherein the elastomeric material is substantially uncrosslinked.

## Patentansprüche

1. Verwendung eines synthetischen elastomeren Polypeptidmaterials, das fähig ist, sich durch einen Umkehrtemperaturübergang zusammenzuziehen und zu entspannen, zur Herstellung einer Barriere zur Verhinderung von Adhäsion zwischen einer Wundstelle in einem Säugetier und einer zweiten Gewebestelle.

2. Verwendung eines Elastomermaterials nach Anspruch 1, worin das Elastomermaterial ein Bioelastomer umfaßt, das elastomere Tetrapeptid- und/oder Pentapeptid-Wiederholungseinheiten aufweist, die hydrophobe Aminosäure- und Glycinreste umfassen, wobei die Wiederholungseinheiten in einer Konformation mit einem β-Turn vorliegen und das Bioelastomer eine Polypepentapeptid-Wiederholungseinheit der Formel:
(R₁PR₂R₃G)ₙ
aufweist, worin R₁ ein peptidbildender Rest von Phe, Leu, Ile oder Val ist; R₂ ein peptidbildender Rest von Ala oder Gly ist; R₃ ein peptidbildender Rest von Phe, Leu, Ile oder Val ist; P ein L-Prolin-bildender Rest ist und G ein Glyzin-bildender Rest ist; und n eine ganze Zahl von 1 bis 5000 ist.

3. Verwendung eines Elastomermaterials nach Anspruch 2, worin R₁ ein peptidbildender Rest von L-Isoleucin oder L-Valin ist; R₂ ein peptidbildender Rest von Glyzin ist; und R₃ ein peptidbildender Rest von L-Valin ist.

4. Verwendung eines Elastomermaterials nach Anspruch 1, worin das Elastomermaterial ein Bioelastomer umfaßt, das elastomere Tetrapeptid- und/oder Pentapeptid-Wiederholungseinheiten aufweist, die hydrophobe Aminosäure- und Glyzinreste umfassen, wobei die Wiederholungseinheiten in einer Konformation mit einem β-Turn vorliegen und das Bioelastomer
(A) eine Pentapeptid-Wiederholungeinheit der Formel IPGVG in einer Polypentapeptideinheit der Formel:
-X¹-(I PGVG)ₙ-Y₁-
und
(B) eine Pentapeptid-Wiederholungseinheit der Formel VPGVG in einer Polypentapeptideinheit der Formel:
-X²-(VPGVG)ₙ-Y²-
aufweist, worin
I ein peptidbildender Rest von L-Isoleucin ist;
P ein peptidbildender Rest von L-Prolin ist;
G ein peptidbildender Rest von Glyzin ist;
V ein peptidbildender Rest von L-Valin ist; und worin X¹ und X² jeweils PGVG, GVG, VG, G oder eine kovalente Bindung sind; Y¹ IPGV, IPG, IP, I oder eine kovalente Bindung ist; und n in beiden Formeln eine ganze Zahl von 1 bis 200 ist oder n 0 ist, X¹ und Y¹ oder X² und Y² gemeinsam zumindest eine der genannten Pentapeptid-Wiederholungseinheiten IPGVG oder VPGVG bilden.

5. Verwendung eines Elastomermaterials nach Anspruch 1, worin das Elastomermaterial ein Bioelastomer umfaßt, das elastomere Tetrapeptid- und/oder Pentapeptid-Wiederholungseinheiten aufweist, die hydrophobe Aminosäure- und Glyzinreste umfassen, wobei die Wiederholungseinheiten in einer Konformation mit einem β-Turn vorliegen und das Bioelastomer eine Polytetrapeptid-Wiederholungseinheit der Formel:
(R₁PGG)ₙ
aufweist, worin R₁ ein peptidbildender Rest von Phe, Leu, Ile oder Val ist; P ein L-Prolin-bildender Rest ist und G ein Glyzin-bildender Rest ist; und n eine ganze Zahl von 1 bis 5000 ist.

6. Verwendung eines Elastomermaterials nach Anspruch 5, worin R₁ ein peptidbildender Rest von L-Valin oder L-Isoleucin ist.

7. Verwendung eines Elastomermaterials nach einem der Ansprüche 2 bis 8, worin das Bioelastomer eine Hexapeptid-Wiederholungseinheit der Formel APGVGV in einer Polyhexapeptideinheit der Formel
-X(APGVGV)ₙY-
aufweist, worin
A ein peptidbildender Rest von L-Alanin ist;
P ein peptidbildender Rest von L-Prolin ist;
G ein peptidbildender Rest von Glyzin ist;
V ein peptidbildender Rest von L-Valin ist;
und X PGVGV, GVGV, VGV, GV, V oder eine kovalente Bindung ist; Y APGVG, APGV, APG, AP, A oder eine kovalente Bindung ist; und n eine ganze Zahl von 2 bis etwa 5000 ist, worin die Einheit zumindest 18 Aminosäurereste enthält.

8. Verwendung nach einem der Ansprüche 1 bis 7, worin das Elastomermaterial im wesentlichen vernetzt ist.

9. Verwendung nach einem der Ansprüche 1 bis 7, worin das Elastomermaterial im wesentlichen unvernetzt ist.

## Revendications

1. Utilisation d'un matériau polypeptidique élastomère synthétique capable de se contracter et se relâcher de façon réversible par une transition de température inverse, pour la fabrication d'une barrière pour la prévention de l'adhérence entre un site de réparation d'une blessure chez un mammifère et un second site du tissu.

2. Utilisation d'un matériau élastomère selon la revendication 1, où le matériau élastomère comprend un bioélastomère qui a des unités récurrentes élastomères tétrapeptidiques et/ou pentapeptidique comprenant des résidus hydrophobes d'acides aminés et de glycine, les unités récurrentes existant sous une conformation ayant une spire β, le bioélastomère ayant une unité récurrente polypentapeptidique de la formule :
(̵R₁PR₂R₃G)̵ₙ
où R₁ est un résidu producteur de peptide de Phe, Leu, Ile ou Val; R₂ est un résidu producteur de peptide de Ala ou Gly; R₃ est un résidu producteur de peptide de Phe, Leu, Ile ou Val; P est un résidu producteur de L-proline et G est un résidu producteur de glycine, et n est un nombre entier de 1 à 5000.

3. Utilisation d'un matériau élastomère selon la revendication 2, où R₁ est un résidu producteur de peptide de L-isoleucine ou de L-valine; R₂ est un résidu producteur de peptide de glycine; et R₃ est un résidu producteur de peptide de L-valine.

4. Utilisation d'un matériau élastomère selon la revendication 1, où le matériau élastomère comprend un bioélastomère qui a des unités récurrentes tétrapeptidiques et/ou pentapeptidique élastomères comprenant des résidus hydrophobes d'acides aminés et de glycine, les unités récurrentes existant sous une conformation en spire β, le bioélastomère ayant
(A) une unité récurrente pentapeptidique de la formule IPGVG dans une unité polypentapeptidique de la formule :
-X¹-(VPGVG)ₙ-Y¹-
et
(B) une unité récurrente pentapeptidique de la formule VPGVG dans une unité polypentapeptidique de la formule :
-X²-(VPGVG)ₙ-Y²-
où
I est un résidu de L-isoleucine formant un peptide;
P est un résidu de L-proline formant un peptide;
G est un résidu de glycine formant un peptide;
V est un résidu de L-valine formant un peptide; et
où chacun de X¹ et X² est PGVG, GVG, VG, G ou une liaison convalente; Y¹ est IPGV, IPG, IP, I ou une liaison convalente; et n dans les deux formules est un nombre entier de 1 à 200 ou bien n est 0, X¹ et Y¹ ou X² et Y² constituant ensemble au moins l'une desdites unités récurrentes pentapeptiques IPGVG ou VPGVG.

5. Utilisation d'un matériau élastomère selon la revendication 1, où le matériau élastomère comprend un bioélastomère qui a des unités récurrentes tétrapeptidiques et/ou pentapeptidiques élastomères comprenant des résidus hydrophobes d'acides aminés et de glycine, les unités récurrentes existant dans une conformation en spire β, le bioélastomère ayant une unité récurrente polytétrapeptidique de la formule :
(̵R₁PGG)̵ₙ
où R1 est un résidu producteur de peptide de Phe, Leu, Ile ou Val; P est un résidu producteur de L-proline et G est un résidu producteur de glycine; et n est un nombre entier de 1 à 5000.

6. Utilisation d'un matériau élastomère selon la revendication 5, où R₁ est un résidu producteur de peptide de L-valine ou de L-isoleucine.

7. Utilisation d'un matériau élastomère selon l'une quelconque des revendications 2 à 8, où le bioélastomère a une unité récurrente hexapeptidique de la formule APGVGV dans une unité polyhexapeptidique de la formule
-X(̵APGVGV)̵ₙY-
où
A est un résidu de L-alanine formant un peptide;
P est un résidu de L-proline formant un peptide;
G est un résidu de glycine formant un peptide;
V est un résidu de L-valine formant un peptide;
et X est PGVGV, GVGV, VGV, GV, V ou une liaison convalente; Y est APGVG, APGV, APG, AP, A ou une liaison convalente; et n est un nombre entier de 2 à environ 5000, où l'unité contient au moins 18 résidus d'acides aminés.

8. Utilisation selon l'une quelconque des revendications 1 à 7, où le matériau élastomère est sensiblement réticulé.

9. Utilisation selon l'une quelconque des revendications 1 à 7, où le matériau élastomère est sensiblement non réticulé.
